# EUROPEAN PATENT APPLICATION

(11) **EP 3 998 043 A2**
(43) Date of publication of application: **18.05.2022**
(21) Application number: 20840019.2
(22) Date of filing: 10.07.2020
(51) Int. Cl.: A61F 2/24, A61B 17/34, A61B 17/00, A61N 1/05

(54) **CERCLAGE OPERATION DEVICE PROVIDED WITH POSITION FIXING DEVICE**

(30) Priority: 12.07.2019 KR 20190084508
(71) Applicant: Tau-PNU Medical Co., Ltd., Busan 46241 (KR)
(72) Inventor: KIM, June Hong, Busan 48516 (KR); PARK, Kyone Peter, Yangsan-si Gyeongsangnam-do 50653 (KR); WON, Yong Hyun, Gimpo-si Gyeonggi-do 10084 (KR)
(74) Representative: dompatent von Kreisler Selting Werner - Partnerschaft von Patent- und Rechtsanwälten mbB
(86) International application number: PCT/KR2020/009127
(87) International publication number: WO 2021/010679

(57) **Abstract**

According to the present disclosure, a cerclage operation device provided with a position fixing device allows the position of the cerclage operation device, which is used in a heart valve or pacemaker implantation operation, to be fixed to prevent movement of the cerclage operation device and maintain the cerclage operation device at the correct position after the operation so that the effect of treatment lasts and the stability of the operation is improved. The cerclage operation device provided with the position fixing device according to the present disclosure includes a cerclage wire, a coronary sinus tube which is inserted into a coronary sinus and has a lumen formed therein, a tricuspid valve tube which crosses a tricuspid valve and has a lumen formed therein, and the position fixing device which is bonded to one side of an outer circumferential surface of the coronary sinus tube and prevents movement of the coronary sinus tube, wherein the coronary sinus tube and the tricuspid valve tube are coupled to each other at an upper side and separated from each other at a lower side.

## Description

### [Technical Field]

The present disclosure relates to a cerclage operation device provided with a position fixing device, and more particularly, a cerclage operation device provided with a position fixing device allowing the position of the cerclage operation device, which is used in a heart valve or pacemaker implantation operation, to be fixed to prevent movement of the cerclage operation device and maintain the cerclage operation device at the correct position after the operation so that the effect of treatment lasts and the stability of the operation is improved.

### [Background Art]

The heart consists of two atriums and two ventricles and is connected to four types of blood vessels including the aorta, vena cava, pulmonary artery, and pulmonary vein to form a passage of blood flow.

Based on the interventricular septum located at the center of the heart, one side is divided into the right atrium and right ventricle, and the other side is divided into the left atrium and left ventricle. The tricuspid valve is present between the right atrium and right ventricle, and the mitral valve is present between the left atrium and left ventricle.

The heart repeatedly contracts and relaxes and serves as a pump such that blood flows along blood vessels. During systole of the heart, blood in the heart flows toward blood vessels. In the right side of the heart, blood flows from the right ventricle to the pulmonary artery, and in the left side of the heart, blood flows from the left ventricle to the aorta.

However, when a valve between an atrium and a ventricle does not function well, blood in the ventricle flows backwards during systole of the heart. When the tricuspid valve between the right atrium and right ventricle does not function properly and blood in the right ventricle flows backwards to the right atrium, it is referred to as "tricuspid regurgitation," and when the mitral valve between the left atrium and left ventricle does not function properly and blood in the left ventricle flows backwards to the left atrium, it is referred to as "mitral regurgitation."

Tricuspid regurgitation often occurs in patients with mitral regurgitation. In patients with heart valve disease, as heart function declines, atrial fibrillation, arrhythmia, heart failure and the like may occur, and a pacemaker may be implanted in the body to treat such conditions.

Accordingly, the inventor of the present disclosure has proposed devices for treating mitral regurgitation, tricuspid regurgitation, heart failure and the like using a cerclage device. More specifically, the inventor of the present disclosure has filed a patent application or been granted a patent for a device for treating mitral regurgitation in Korean Patent Registration No. 10-1116867, Korean Patent Registration No. 10-1231140, Korean Patent Registration No. 10-1563172, Korean Patent Registration No. 10-1581021, and Korean Unexamined Patent Application Publication No. 10-2013-0074823, has filed a patent application or been granted a patent for a device for treating tricuspid regurgitation in Korean Patent Registration No. 10-1805679 and Korean Unexamined Patent Application Publication No. 10-2015-0144568, and has filed a patent application for a device for placing an end of a pacemaker lead in Korean Unexamined Patent Application Publication No. 10-2016-0011530 and Korean Unexamined Patent Application Publication No. 10-2016-0020887.

The above patents basically relate to a "cerclage device" including a coronary sinus tube which is inserted into a coronary sinus, a tricuspid valve tube which crosses a tricuspid valve, and a cerclage wire. The "cerclage device" is a name given in the sense that the cerclage wire is inserted into the superior vena cava, coronary sinus, interventricular septum, right atrium, and superior vena cava and connected thereto in a circle in the body. The cerclage wire is inserted into the coronary sinus tube and the tricuspid valve tube, and both ends of the cerclage wire pass through the superior vena cava in a circle in the body and are located outside the body.

In the "cerclage device," the coronary sinus tube and the tricuspid valve tube are coupled to each other at an upper portion and are separated from each other at a lower portion, and the portion where the coronary sinus tube and the tricuspid valve tube are separated is located at an entrance of the coronary sinus.

Here, in the "cerclage device," a case where the position of the coronary sinus tube is moved due to sliding toward the coronary sinus after the operation often occurs, and the present disclosure improves the "cerclage device" to address such a problem.

### [Disclosure]

### [Technical Problem]

The present disclosure is directed to providing a cerclage device provided with a position fixing device that is not only capable of fixing the position of the device so that the position does not move after the operation, but also capable of providing the device which does not cause damage to the body and is easy to fix and detach, thus maintaining the device at the correct position and improving the stability of the operation.

The objectives of the present disclosure are not limited to those mentioned above, and other unmentioned objectives should be clearly understood by those of ordinary skill in the art from the description below.

### [Technical Solution]

The present disclosure provides a cerclage operation device provided with a position fixing device, the cerclage operation device including a cerclage wire, a coronary sinus tube which is inserted into a coronary sinus and has a lumen formed therein, a tricuspid valve tube which crosses a tricuspid valve and has a lumen formed therein, and the position fixing device which is bonded to one side of an outer circumferential surface of the coronary sinus tube and prevents movement of the coronary sinus tube, wherein the coronary sinus tube and the tricuspid valve tube are coupled to each other at an upper side and separated from each other at a lower side.

The position fixing device may include a coupling portion which is formed in a quadrilateral shape and coupled to surround the outer circumferential surface of the coronary sinus tube and one or more fixing portions which are formed to extend from a lower end of the coupling portion and are tilted at a predetermined angle toward the tricuspid valve tube so as to be easily inserted when being inserted into an inner wall of the coronary sinus and easily detached when being detached therefrom, wherein the fixing portion is fixed to the inner wall of the coronary sinus and not fixed to an outer wall of the coronary sinus that is thinner than the inner wall of the coronary sinus, and thus damage to the coronary sinus is minimized.

According to another embodiment, the position fixing device may include a coupling portion which is formed in a quadrilateral shape and coupled to surround the outer circumferential surface of the coronary sinus tube, one or more first fixing portions which are formed to extend from a lower end of the coupling portion and are tilted at a predetermined angle toward the tricuspid valve tube so as to be easily inserted when being inserted into an inner wall of the coronary sinus and easily detached when being detached therefrom, and one or more second fixing portions which are formed to extend from an upper surface of a groove formed in one side of the coupling portion and are tilted at a predetermined angle toward the tricuspid valve tube so as to be easily inserted when being inserted into the inner wall of the coronary sinus and easily detached when being detached therefrom, wherein the first fixing portion and the second fixing portion are fixed to the inner wall of the coronary sinus and not fixed to an outer wall of the coronary sinus that is thinner than the inner wall of the coronary sinus, and thus damage to the coronary sinus is minimized.

According to yet another embodiment of the present disclosure, the position fixing device may include a coupling portion which is formed in a quadrilateral shape and coupled to surround the outer circumferential surface of the coronary sinus tube and a fixing portion which is formed to protrude outward from one side surface of the coupling portion along a lower outer circumferential surface thereof, wherein the fixing portion is formed with a predetermined slope from an upper end to a lower end so as to protrude the most at the lower end.

According to still another embodiment of the present disclosure, the position fixing device may include a coupling portion which is formed in a quadrilateral shape and coupled to surround the outer circumferential surface of the coronary sinus tube and a plurality of fixing portions which are arranged in a plurality of rows and columns on one side surface of the coupling portion and are each formed in the shape of a microneedle.

According to an exemplary embodiment of the present disclosure, the tricuspid valve tube may include a blocking portion which is coupled to one side to block an orifice of the tricuspid valve.

Also, the tricuspid valve tube may include a stopper which is coupled to an end to prevent the tricuspid valve tube from digging into the interventricular septum.

In addition, the cerclage wire may include an arch portion coupled to one side to protect a coronary artery located below the coronary sinus from being pressed by the cerclage wire.

The position fixing device may be fixed to the inner wall of the coronary sinus and not fixed to an outer wall of the coronary sinus that is thinner than the inner wall of the coronary sinus, and thus damage to the coronary sinus is minimized.

Also, the position fixing device may be provided as one or more position fixing devices coupled along the coronary sinus tube.

### [Advantageous Effects]

A cerclage device provided with a position fixing device according to the present disclosure allows the position fixing device to be fixed to an inner wall of a coronary sinus when tension is applied to an operation wire and prevents the position of a coronary sinus tube from moving due to sliding after the operation, thus having advantages in that the stability of the operation is improved, the device is maintained at the correct position inside the body after the operation so that the effect of treatment is improved, and reoperation due to the movement of the position of the cerclage device is not required.

### [Description of Drawings]

FIG. 1 is a perspective view of a cerclage device provided with a position fixing device according to the present disclosure.
FIG. 2A is a picture showing a state of operation when the cerclage device provided with the position fixing device according to the present disclosure is inserted into the heart, and FIG. 2B is a perspective view illustrating a coronary sinus.
FIG. 3 is a perspective view of the position fixing device according to a first exemplary embodiment of the present disclosure, wherein FIG. 3A is a perspective view when the position fixing device is unfolded, and FIG. 3B is a perspective view when the position fixing device is coupled.
FIG. 4 is a perspective view of the position fixing device according to a second exemplary embodiment of the present disclosure.
FIG. 5 is a perspective view of the position fixing device according to a third exemplary embodiment of the present disclosure.
FIG. 6 is a perspective view of the position fixing device according to a fourth exemplary embodiment of the present disclosure.
FIG. 7 is a lateral view of the position fixing device according to a fifth exemplary embodiment of the present disclosure.
FIG. 8 is a perspective view of the position fixing device according to a sixth exemplary embodiment of the present disclosure.

### <Description of reference numerals>

1: cerclage wire
10: coronary sinus tube
20: tricuspid valve tube
30: position fixing device
32: coupling portion
32-1: protruding portion
32-2: first recessed portion
32-3: second recessed portion
34: fixing portion
36: groove
37: first fixing portion
38: second fixing portion
40: blocking portion
50: stopper
60: arch portion

### [Modes of the Invention]

The advantages and features of the present disclosure and methods of achieving the same will become more apparent by referring to embodiments described in detail below with reference to the accompanying drawings. However, the present disclosure is not limited to the embodiments disclosed below and may be implemented in various different forms. The present embodiments make the disclosure of the present disclosure complete and are provided to completely inform those of ordinary skill in the art to which the present disclosure pertains of the scope of the present disclosure. The present disclosure is only defined by the scope of the claims.

Details for embodying the present disclosure will be described in detail with reference to the accompanying drawings. The same reference numerals refer to the same elements regardless of the drawings, and the term "and/or" may refer to each mentioned item or any combination of one or more of the mentioned items.

The terms used herein are for describing the embodiments and are not intended to limit the present disclosure. In the present specification, a singular expression includes a plural expression unless the context clearly indicates otherwise. The terms "comprises" and/or "comprising" used herein do not preclude the presence or addition of one or more elements other than those mentioned.

Unless defined otherwise, all terms including technical or scientific terms used herein may have the same meaning as commonly understood by those of ordinary skill in the art to which the present disclosure pertains. Also, terms defined in commonly used dictionaries should not be construed in an idealized or overly formal sense unless expressly so defined herein.

Hereinafter, exemplary embodiments of the present disclosure will be described in detail with reference to the accompanying drawings.

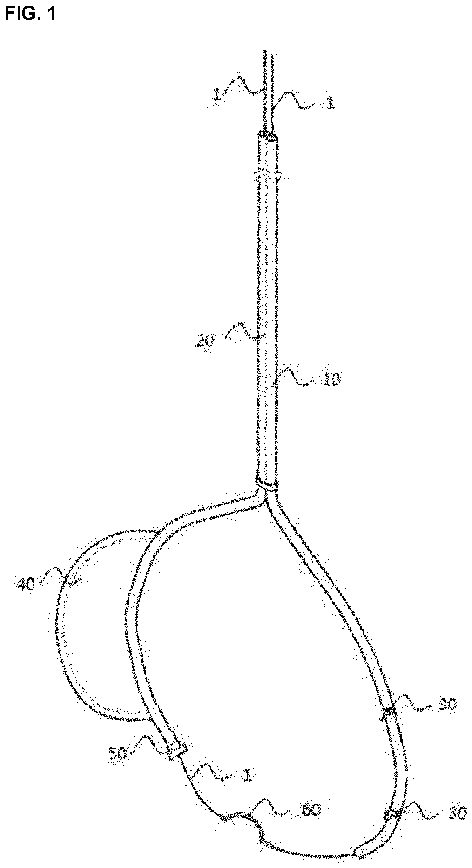

FIG. 1 is a perspective view of a cerclage device provided with a position fixing device according to the present disclosure.

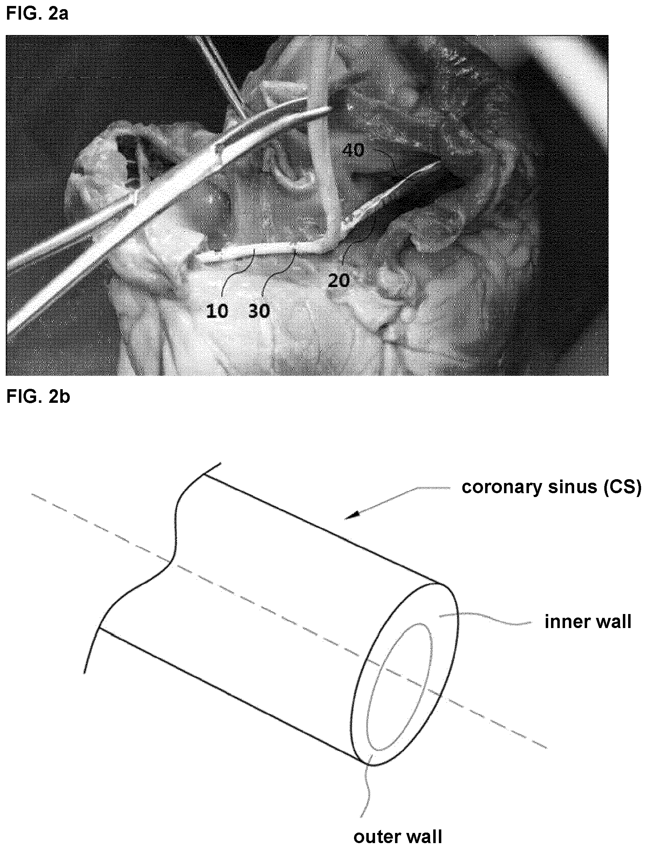

FIG. 2A is a picture showing a state of operation when the cerclage device provided with the position fixing device according to the present disclosure is inserted into the heart, and FIG. 2B is a perspective view illustrating a coronary sinus.

Referring to FIGS. 1 and 2, the cerclage device provided with the position fixing device according to the present disclosure includes a cerclage wire 1, a coronary sinus tube 10, a tricuspid valve tube 20, and a position fixing device 30.

The cerclage wire 1 is a name given in the sense that it is connected to the coronary sinus, interventricular septum, and tricuspid valve in a circle. As illustrated in the drawing, the cerclage wire 1 is inserted into the coronary sinus tube 10 and the tricuspid valve tube 20 and connected thereto in one circle.

An arch portion 60 is formed at one side of the cerclage wire 1. The cerclage wire 1 is inserted into the coronary sinus, and a coronary artery is located below the coronary sinus. Here, considerable external pressure is applied to the coronary artery by the cerclage wire 1. The arch portion 60 prevents this and protects the coronary artery.

The coronary sinus tube 10 has a lumen formed therein and has a lower portion inserted into the coronary sinus to protect the coronary sinus. The coronary sinus tube 10 may be made of a material, such as rubber or flexible plastic, that has high ductility and has high flexibility and restorability to allow the coronary sinus tube 10 to move according to the beating of the heart.

The tricuspid valve tube 20 has a lumen formed therein and passes through an orifice, which is formed due to incomplete closing of the tricuspid valve, to reach the interventricular septum. The tricuspid valve tube 20 may be made of a material, such as rubber or flexible plastic, that has high ductility and has high flexibility and restorability to allow the tricuspid valve tube 20 to move according to the beating of the heart.

The tricuspid valve tube 20 includes a blocking portion 40 coupled to a side surface. The blocking portion 40 blocks the orifice formed due to the incomplete closing of the tricuspid valve, and a blocking membrane, a blocking balloon, or the like may be used as the blocking portion 40. The blocking portion 40 blocks the orifice of the tricuspid valve to treat tricuspid regurgitation.

In order to prevent an end of the tricuspid valve tube 20 from penetrating into the interventricular septum, a stopper 50 is installed at a lower end of the tricuspid valve tube 20. This allows the tricuspid valve tube 20 to float instead of coming in close contact with a portion around the tricuspid valve to prevent damage to the tricuspid valve due to the tricuspid valve tube 20.

The position fixing device 30 is bonded to one side of an outer circumferential surface of the coronary sinus tube 10 at one side of the coronary sinus tube 10. One or more position fixing devices 30 are bonded to prevent the coronary sinus tube 10 from sliding or moving due to the beating of the heart.

FIG. 2B is a view for describing the coronary sinus in more detail. The coronary sinus is formed such that the inner wall is thicker than the outer wall. The position fixing device 30 is fixed to the inner wall of the coronary sinus and is not fixed to the outer wall of the coronary sinus that is thinner than the inner wall of the coronary sinus, and thus damage to the coronary sinus is minimized.

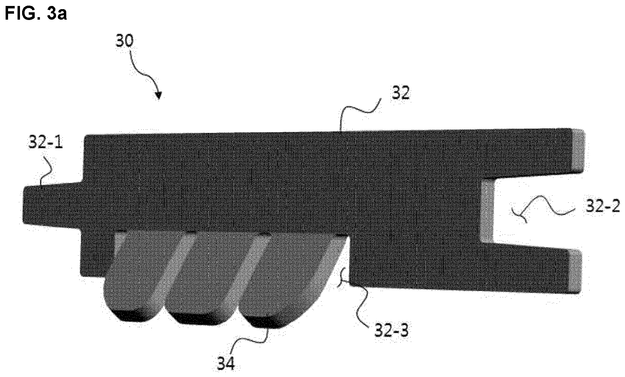

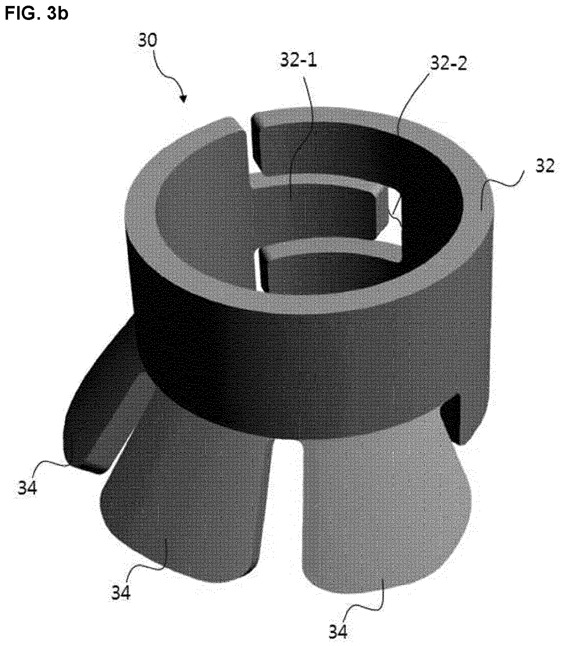

The position fixing device 30 will be described in more detail below with reference to FIGS. 3 to 8.

Also, the cerclage device provided with the position fixing device according to the present disclosure may include a defibrillator lead (not illustrated) inserted into the coronary sinus tube 10 or the tricuspid valve tube 20 to treat atrial fibrillation or a pacemaker lead (not illustrated) whose end is inserted into the bundle of His to apply electrical stimulation to the heart.

FIG. 3 is a perspective view of the position fixing device according to a first exemplary embodiment of the present disclosure, wherein FIG. 3A is a perspective view when the position fixing device is unfolded, and FIG. 3B is a perspective view when the position fixing device is coupled.

The position fixing device 30 according to the first exemplary embodiment of the present disclosure will be described in more detail with reference to FIG. 3. The position fixing device 30 includes a coupling portion 32 and a fixing portion 34.

The coupling portion 32 has a quadrilateral shape and surrounds the coronary sinus tube 10. The coupling portion 32 includes a protruding portion 32-1 formed to protrude to extend from one end of the coupling portion 32 in the left-right direction, a first recessed portion 32-2 recessed toward the one end from the other end of the coupling portion 32 in the left-right direction and formed to correspond to the shape of the protruding portion 32-1, and a second recessed portion 32-3 recessed toward an upper end from a lower end of the coupling portion 32.

The coupling portion 32 is coupled to surround the outer circumferential surface of the coronary sinus tube 10 in a state in which the protruding portion 32-1 is inserted into the first recessed portion 32-2, and after being coupled, the coupling portion 32 has a ring shape as a whole. When coupling the coupling portion 32 to the coronary sinus tube 10, an adhesive material such as an adhesive harmless to the human body is applied for adhesion.

Both ends of the coupling portion 32 in the left-right direction at which the protruding portion 32-1 and the first recessed portion 32-2 are formed may be coupled at positions where the ends come in contact with the outer wall of the coronary sinus.

The position fixing device 30 includes a plurality of fixing portions 34 which are formed to extend from an upper end of the second recessed portion 32-3 and are tilted at a predetermined angle toward the tricuspid valve tube so as to be easily inserted when being inserted into the inner wall of the coronary sinus and easily detached when being detached therefrom. The fixing portion 34 has one end coming in contact with an upper surface of the second recessed portion 32-3 and the other end protruding toward the tricuspid valve tube 20, has a quadrilateral shape whose length in a width direction gradually increases from the one end toward the other end, and is formed to be rounded without angular corners.

More specifically, the fixing portion 34 is located to come in contact with the inner wall of the coronary sinus, and when the coronary sinus tube 10 is inserted into the coronary sinus, the fixing portion 34 is fixed to the inner wall of the coronary sinus.

The inner wall of the coronary sinus is formed thicker than the outer wall thereof. Thus, the inner wall of the coronary sinus is not damaged even when the fixing portion 34 is fixed to the inner wall of the coronary sinus, but in a case where the fixing portion 34 is fixed to the outer wall of the coronary sinus, the outer wall of the coronary sinus may be torn or damaged due to its small thickness.

Therefore, the position fixing device 30 according to the present disclosure is coupled to the coronary sinus tube 10, and the fixing portion 34 is fixed to the inner wall of the coronary sinus, and in this way, sliding or moving of the coronary sinus tube 10 is prevented, but damage is not caused to the coronary sinus.

Also, since both side corners of the fixing portion 34 are formed to be rounded, the fixing portion 34 may fix the position of the coronary sinus tube 10 without causing deep damage to the inner wall of the coronary sinus even when fixed to the inner wall of the coronary sinus.

The coupling portion 32 may also have a ring shape that does not include the first recessed portion 32-2 and the protruding portion 32-1. Alternatively, the coupling portion 32 may not include the second recessed portion 32-3 and may include the fixing portion 34 formed to directly extend from a lower end of the coupling portion 32.

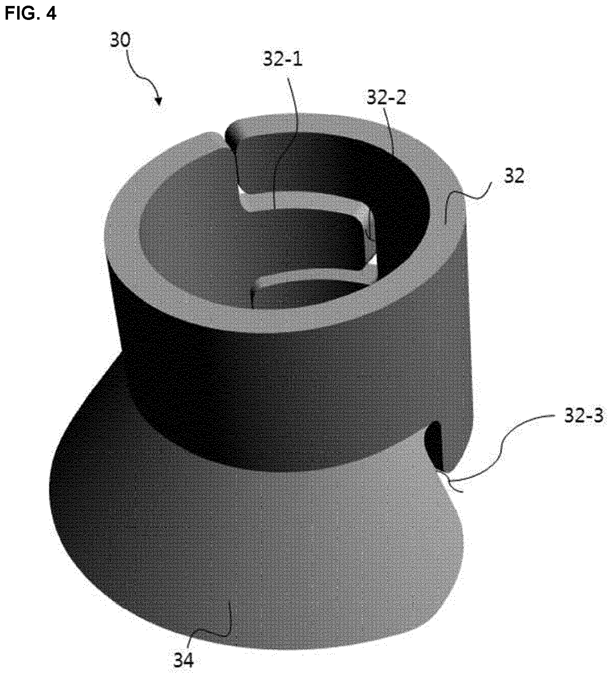

FIG. 4 is a perspective view of the position fixing device according to a second exemplary embodiment of the present disclosure.

The position fixing device 30 according to the second exemplary embodiment of the present disclosure includes a coupling portion 32 and a fixing portion 34. The coupling portion 32 is the same as that described above with reference to FIG. 3, and the number of fixing portions 34 coupled to the coupling portion 32 is different in the position fixing device 30 according to the second exemplary embodiment of the present disclosure.

In the position fixing device 30 according to the second embodiment, a single fixing portion 34 is formed to extend from the upper surface of the second recessed portion 32-3 and is formed to be tilted at a predetermined angle toward the tricuspid valve tube so as to be easily inserted when being inserted into the inner wall of the coronary sinus and easily detached when being detached therefrom. More specifically, the fixing portion 34 has one end coming in contact with the upper surface of the second recessed portion 32-3 and the other end protruding toward the tricuspid valve tube, has a quadrilateral shape whose length in a width direction gradually increases from the one end toward the other end, and is formed to be rounded without angular corners.

The fixing portion 34 of the position fixing device 30 according to the second exemplary embodiment of the present disclosure is formed to be larger than the fixing portion 34 according to the first embodiment. Therefore, a fixing strength is excellent even when only one fixing portion 34 is coupled.

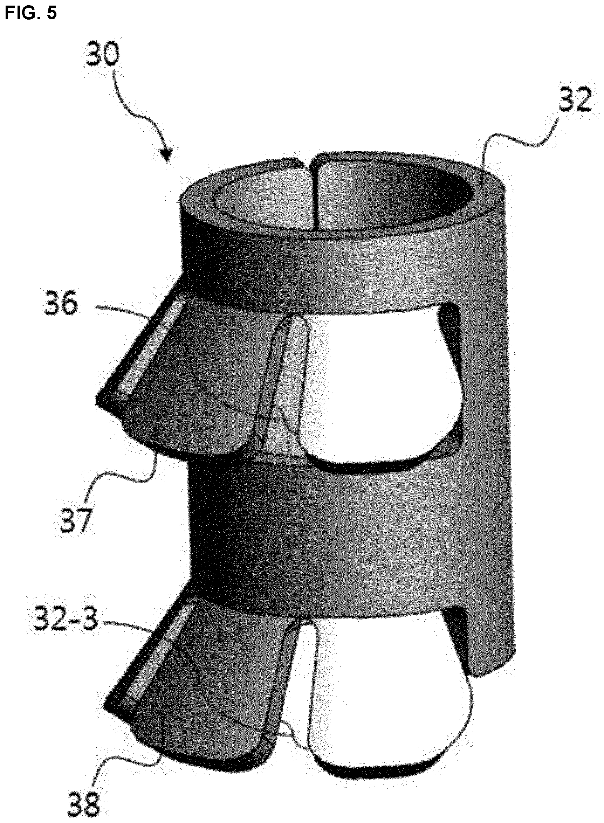

FIG. 5 is a perspective view of the position fixing device according to a third exemplary embodiment of the present disclosure. The position fixing device 30 according to the third exemplary embodiment of the present disclosure includes a coupling portion 32 and a first fixing portion 37. The coupling portion 32 and the first fixing portion 37 have the same shape as the coupling portion and the fixing portion described above with reference to FIG. 3, but the length of the coupling portion 32 in the height direction is formed to be longer than that illustrated in FIG. 3.

A quadrilateral groove 36 is formed to be horizontal to the second recessed portion 32-2 at one side of the coupling portion 32, and the position fixing device 30 includes a plurality of second fixing portions 38 which are formed to extend from an upper surface of the groove 36 and are bent at a predetermined angle toward the tricuspid valve tube 20. The second fixing portion 38 is formed to be the same as the fixing portion 34, and a plurality of second fixing portions 38 are formed to be spaced apart from each other at predetermined intervals.

In the position fixing device 30 according to the third exemplary embodiment of the present disclosure, the first fixing portion 37 and the second fixing portion 38 are formed in a single position fixing device 30. Thus, a fixing strength may be improved even when the single position fixing device 30 is bonded to the coronary sinus tube 10.

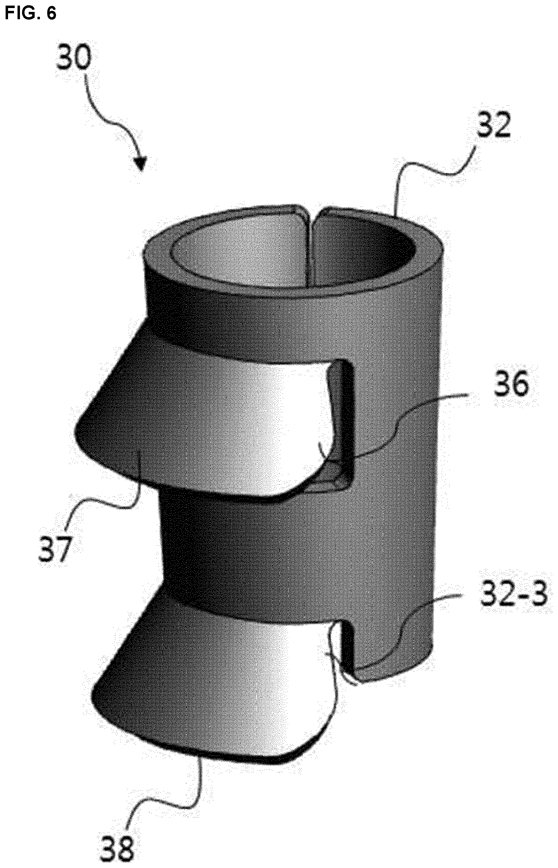

FIG. 6 is a perspective view of the position fixing device according to a fourth exemplary embodiment of the present disclosure. The position fixing device 30 according to the fourth exemplary embodiment of the present disclosure includes a coupling portion 32 and a first fixing portion 37, includes a quadrilateral groove 36 formed to be horizontal to a second recessed portion 32-3 at one side of the coupling portion 32, and includes a single second fixing portion 38 which is formed to extend from an upper surface of the groove 36 and bent at a predetermined angle toward the tricuspid valve tube 20.

The coupling portion 32 and the groove 36 are the same as those described above with reference to FIG. 5, and the first fixing portion 37 includes a single fixing portion 34 which is formed to extend from an upper end of the second recessed portion 32-3 and are tilted at a predetermined angle toward the tricuspid valve tube so as to be easily inserted when being inserted into the inner wall of the coronary sinus and easily detached when being detached therefrom.

More specifically, the first fixing portion 37 and the second fixing portion 38 have the same form as the fixing portion 34 according to the second exemplary embodiment of the present disclosure described above with reference to FIG. 4, the first fixing portion 37 and the second fixing portion 38 are formed to extend from the second recessed portion 32-3 and the groove 36, respectively, and protrude to be bent at a predetermined angle toward the tricuspid valve tube 20, have a quadrilateral shape whose length in a width direction gradually increases form one end toward the other end, and are formed to be rounded without angular corners.

In the position fixing device 30 according to the fourth exemplary embodiment of the present disclosure, the two fixing portions 37 and 38 are formed in a single position fixing device 30. Thus, a fixing strength may be further improved even when only the single position fixing device 30 is coupled to the coronary sinus tube 10.

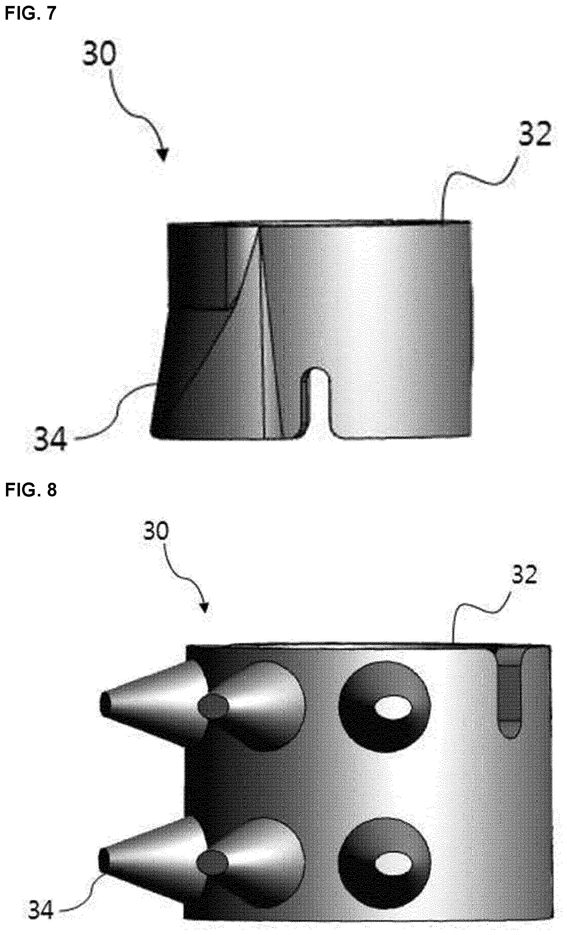

FIG. 7 is a lateral view of the position fixing device according to a fifth exemplary embodiment of the present disclosure. The position fixing device 30 according to the fifth embodiment of the present disclosure includes a coupling portion 32 and a fixing portion 34. The coupling portion 32 includes a protruding portion 32-1 and a first recessed portion 32-2 which are formed to be the same as those described above with reference to FIG. 3 but does not include a second recessed portion 32-3.

The fixing portion 34 is formed to protrude outward along a lower outer circumferential surface of the coupling portion 32 located to face the tricuspid valve tube 20. More specifically, the fixing portion 34 is formed with a predetermined slope from an upper end to a lower end so as to protrude the most at the lower end. When the coronary sinus tube 10 is inserted into the coronary sinus, the lower end of the fixing portion 34 is caught in one side of the inner wall of the coronary sinus and serves as a bump that prevents further sliding of the coronary sinus tube 10.

The coupling portion 32 may have a groove formed at each of left and right sides to which the fixing portion 34 is coupled, and the grooves allow the coupling portion 32 to bend flexibly and be coupled to the coronary sinus tube 10.

FIG. 8 is a perspective view of the position fixing device according to a sixth exemplary embodiment of the present disclosure. The position fixing device 30 according to the sixth embodiment of the present disclosure includes a coupling portion 32 and a fixing portion 34. The coupling portion 32 includes a protruding portion 32-1 and a first recessed portion 32-2 which are formed to be the same as those described above with reference to FIG. 3 but does not include a second recessed portion 32-3.

A plurality of fixing portions 34 coupled to an outer circumferential surface of the coupling portion 32 are included, and the fixing portions 34 are each formed in a conical shape and are arranged in a plurality of rows and columns on one side of the coupling portion 32 that faces the tricuspid valve tube 20. The fixing portion 34 is formed in the shape of a microneedle. More specifically, microneedles of various shapes such as a conical shape, a polygonal pyramidal shape, and a spiral shape that have a size less than 1 mm may be applied.

More specifically, when the cerclage wire 1 is pulled, the fixing portion 34 is fixed to the inner wall of the coronary sinus and fixes the position of the coronary sinus tube 10, but when the tension on the cerclage wire 1 is released, the fixing portion 34 is easily detached from the inner wall of the coronary sinus and does not cause damage to the inner wall of the coronary sinus.

A method of operation using a cerclage operation device provided with a position fixing device according to the present disclosure will be briefly described. First, the cerclage operation device is inserted into the body. Here, the cerclage operation device basically includes a coronary sinus tube 10 and a tricuspid valve tube 20, and one or more position fixing devices 30 are coupled to one side of the coronary sinus tube 10. Also, a stopper 50 may be included on a lower end of the tricuspid valve tube 20, and a blocking portion 40 may be included on one side of an outer circumferential surface of the tricuspid valve tube 20.

Next, the cerclage wire 1 is inserted into the superior vena cava, the coronary sinus tube 10, the interventricular septum, and the tricuspid valve tube 20 and caused to protrude from the superior vena cava again. Here, an arch portion 60 may be included on one side of the cerclage wire 1.

Next, when both ends of the cerclage wire 1 located at the superior vena cava are pulled, tension is applied to the cerclage wire 1, the coronary sinus tube 10 is moved toward the inner wall of the coronary sinus, and the position fixing device 30 is fixed to the inner wall of the coronary sinus.

Therefore, the cerclage operation device is prevented from sliding or moving due to the beating of the heart, thus improving the stability of the operation and allowing the device to be fixed and maintained at the correct position inside the body.

The embodiments of the present disclosure have been described above with reference to the accompanying drawings, but those of ordinary skill in the art to which the present disclosure pertains should understand that the present disclosure may be embodied in other specific forms without changing the technical idea or essential features of the present disclosure. Therefore, the embodiments described above should be understood as being illustrative, instead of limiting, in all aspects.

## Claims

1. A cerclage operation device provided with a position fixing device, the cerclage operation device comprising:
a cerclage wire;
a coronary sinus tube which is inserted into a coronary sinus and has a lumen formed therein;
a tricuspid valve tube which crosses a tricuspid valve and has a lumen formed therein; and
the position fixing device which is bonded to one side of an outer circumferential surface of the coronary sinus tube and prevents movement of the coronary sinus tube,
wherein the coronary sinus tube and the tricuspid valve tube are coupled to each other at an upper side and separated from each other at a lower side.

2. The cerclage operation device of claim 1, wherein the position fixing device includes:
a coupling portion which is formed in a quadrilateral shape and coupled to surround the outer circumferential surface of the coronary sinus tube; and
one or more fixing portions which are formed to extend from a lower end of the coupling portion and are tilted at a predetermined angle toward the tricuspid valve tube so as to be easily inserted when being inserted into an inner wall of the coronary sinus and easily detached when being detached therefrom,
wherein the fixing portion is fixed to the inner wall of the coronary sinus and not fixed to an outer wall of the coronary sinus that is thinner than the inner wall of the coronary sinus, and thus damage to the coronary sinus is minimized.

3. The cerclage operation device of claim 1, wherein the position fixing device includes:
a coupling portion which is formed in a quadrilateral shape and coupled to surround the outer circumferential surface of the coronary sinus tube;
one or more first fixing portions which are formed to extend from a lower end of the coupling portion and are tilted at a predetermined angle toward the tricuspid valve tube so as to be easily inserted when being inserted into an inner wall of the coronary sinus and easily detached when being detached therefrom; and
one or more second fixing portions which are formed to extend from an upper surface of a groove formed in one side of the coupling portion and are tilted at a predetermined angle toward the tricuspid valve tube so as to be easily inserted when being inserted into the inner wall of the coronary sinus and easily detached when being detached therefrom,
wherein the first fixing portion and the second fixing portion are fixed to the inner wall of the coronary sinus and not fixed to an outer wall of the coronary sinus that is thinner than the inner wall of the coronary sinus, and thus damage to the coronary sinus is minimized.

4. The cerclage operation device of claim 1, wherein the position fixing device includes:
a coupling portion which is formed in a quadrilateral shape and coupled to surround the outer circumferential surface of the coronary sinus tube; and
a fixing portion which is formed to protrude outward from one side surface of the coupling portion along a lower outer circumferential surface thereof,
wherein the fixing portion is formed with a predetermined slope from an upper end to a lower end so as to protrude the most at the lower end.

5. The cerclage operation device of claim 1, wherein the position fixing device includes:
a coupling portion which is formed in a quadrilateral shape and coupled to surround the outer circumferential surface of the coronary sinus tube; and
a plurality of fixing portions which are arranged in a plurality of rows and columns on one side surface of the coupling portion and are each formed in the shape of a microneedle.

6. The cerclage operation device of claim 1, wherein the tricuspid valve tube includes a blocking portion which is coupled to one side to block an orifice of the tricuspid valve.

7. The cerclage operation device of claim 1, wherein the tricuspid valve tube includes a stopper which is coupled to an end to prevent the tricuspid valve tube from digging into the interventricular septum.

8. The cerclage operation device of claim 1, wherein the cerclage wire includes an arch portion coupled to one side to protect a coronary artery located below the coronary sinus from being pressed by the cerclage wire.

9. The cerclage operation device of claim 1, wherein the position fixing device is fixed to the inner wall of the coronary sinus and not fixed to an outer wall of the coronary sinus that is thinner than the inner wall of the coronary sinus, and thus damage to the coronary sinus is minimized.

10. The cerclage operation device of claim 1, wherein the position fixing device is provided as one or more position fixing devices coupled along the coronary sinus tube.
